# DEMANDE DE BREVET EUROPEEN

(11) **EP 0 548 437 A1**
(43) Date de publication de la demande: **30.06.1993**
(21) Numéro de dépôt: 91460059.8
(22) Date de dépôt: 20.12.1991
(51) Int. Cl.: A01K 67/033

(54) **Procédé d'incubation d'oeufs d'escargots, boîte d'incubation, enceintes d'incubation et de nursery pour la mise en oeuvre de ce procédé**

(71) Demandeur: ETUDES DE MACHINES SPECIALES DE L'OUEST, F-35740 Pace (FR)
(72) Inventeur: Saulnier, Michel, F-35760 Montgermont (FR)
(74) Mandataire: Vidon, Patrice

(57) **Abrégé**

La présente invention concerne un procédé d'incubation d'oeufs d'escargots (O), qui consiste à séparer les oeufs (O) du substrat (5) dans lequel ils ont été pondus, à les placer dans des boîtes d'incubation (2) dont au moins une grande face (200) présente des ouvertures de dimensions plus petites que le diamètre des oeufs, à placer lesdites boîtes à proximité immédiate d'une eau (E) à niveau constant, la face (200) présentant des ouvertures étant tournée vers ladite eau, puis, lorsque les oeufs sont éclos, à ouvrir lesdites boîtes (2) et à les assembler avec une enceinte de nursery (8) dépourvue de fond, de telle sorte que les grandes faces (200) des boîtes forment, après assemblage, un fond (82) pour l'enceinte de nursery (8). L'invention concerne également une boîte d'incubation, ainsi que des enceintes d'incubation et de nursery pour la mise en oeuvre de ce procédé.

## Description

La présente invention est relative à l'élevage des escargots.

Elle concerne plus particulièrement un procédé d'incubation d'oeufs d'escargots ainsi que des boîte et enceinte d'incubation et une enceinte de nursery pour la mise en oeuvre de ce procédé.

Diverses techniques d'élevages d'escargots sont bien connues. Ainsi, on a proposé de mettre en oeuvre la phase d'incubation des oeufs d'escargots en laissant ceux-ci dans les pots dans lesquels ils ont été pondus jusqu'à leur éclosion, ceci sous atmosphère contrôlée. Cette technique présente des difficultés liées aux paramètres de température et d'hygrométrie favorables à l'éclosion des oeufs, paramètres difficiles à respecter. Par conséquent, l'éclosion des oeufs n'est pas contrôlée dans le temps, c'est-à-dire qu'elle peut différer de plusieurs jours selon les individus. De plus, une telle technique provoque une mortalité élevée des escargots avant ou après l'éclosion des oeufs.

Par ailleurs, on connait des techniques d'élevage hors sol consistant à changer les escargots d'enceinte d'élevage au fur et à mesure de leur développement. Ainsi, on transfère les escargots dans une enceinte de plus grande dimension lorsque leur taille s'est accrue. Il est alors nécessaire de manipuler les escargots. Ceci est relativement gênant car ces individus surtout lorsqu'ils sont jeunes sont très sensibles aux germes et bactéries que peuvent leur apporter les opérateurs lors de la manipulation, ce qui entraîne une mortalité élevée.

La présente invention a notamment pour but de pallier les inconvénients cités ci-dessus.

A ce titre, elle propose un procédé d'incubation qui permet d'opérer un meilleur contrôle des paramètres favorables à l'éclosion des oeufs, tout en réduisant les risques de mortalité.

Ce procédé permet d'opérer un transfert des jeunes escargots dans une enceinte de nursery sans avoir à les manipuler individuellement, ce qui permet également de réduire très fortement le taux de mortalité lors de l'élevage.

Il s'agit d'un procédé simple à mettre en oeuvre, ceci avec du matériel peu onéreux.

Ce procédé d'incubation d'oeufs d'escargots consiste à séparer les oeufs du substrat dans lequel ils ont été pondus, à les placer dans des boîtes d'incubation dont au moins une grande face présente des ouvertures de dimensions plus petites que le diamètre des oeufs, à placer les boîtes à proximité immédiate d'une eau à niveau constant, la face présentant des ouvertures étant tournée vers ladite eau, puis, lorsque les oeufs sont éclos, à ouvrir lesdites boîtes et à les assembler avec une enceinte de nursery dépourvue de fond, de telle sorte que les grandes faces des boîtes forment après assemblage un fond pour l'enceinte de nursery.

De préférence, lorsque l'on place les boîtes à proximité de ladite eau, la distance qui sépare la face de la boîte tournée vers l'eau de cette dernière est d'environ 1 mm.

On notera, que lorsque les oeufs sont éclos, ceux-ci sont transférés dans l'enceinte de nursery sans que l'opérateur ait à les manipuler, ce qui évite le stress des individus et une contamination de ceux-ci par certains germes ou bactéries apportés par le manipulateur.

L'invention concerne également une boîte d'incubation pour la mise en oeuvre de ce procédé.

Selon une caractéristique avantageuse, cette boîte consiste en deux éléments identiques formant demi-boîtes, lesquelles comportent une grande face de forme carrée pourvue, sur deux de ses côtés opposés et perpendiculairement à celle-ci, d'une paroi latérale.

De préférence, ladite paroi latérale est chanfreinée sur son côté tourné vers ladite grande face.

Par ailleurs, au moins une partie de la surface de ladite grande face est formée d'un treillis.

L'invention concerne également une enceinte d'incubation qui peut être utilisée pour la mise en oeuvre du procédé.

De préférence, cette enceinte a la forme d'un bac qui est équipée de moyens de circulation d'eau et de maintien à niveau de celle-ci, l'enceinte étant pourvue également de moyens supports pour les boîtes d'incubation.

Selon une caractéristique avantageuse, les moyens de maintien a niveau de l'eau consistent en un conduit sensiblement vertical dont l'orifice supérieur débouche à une certaine distance du fond du bac et qui assure l'évacuation du trop plein d'eau.

Selon un mode de réalisation préférentiel les moyens supports consistent en des châssis de forme rectangulaire sur lesquels les boîtes d'incubation sont destinées à venir reposer par deux de leurs côtés opposés et qui s'étendent parallèlement à l'un des côtés de l'enceinte, ces cadres étant disposés de telle façon que, lorsque les boîtes sont en place, celles-ci se trouvent à proximité immédiate de l'eau.

De manière avantageuse, les cadres reposent sur des tiges supports.

De préférence, l'enceinte est pourvue de moyens de fermeture hermétiques.

Enfin, l'invention concerne également une enceinte de nursery pour la mise en oeuvre du procédé.

Selon une caractéristique particulièrement avantageuse, cette enceinte est formée d'une boîte qui comporte un fond ouvert de forme rectangulaire et les chants des deux grandes faces opposées de cette boîte adjacents au fond ouvert présentent une rainure de forme complémentaire de celle des parois latérales de la boîte d'incubation décrite ci-dessus, les dimensions du fond ouvert étant telles que l'on peut mettre en place côte à côte et par glissement dans les rainures, plusieurs demi-boîtes constituant les boîtes d'incubation, de façon à constituer un fond pour l'enceinte de nursery.

Selon une autre caractéristique, les parois de cette enceinte ne comportent aucun angle vif.

De préférence, cette enceinte est formée, au moins en partie, d'un treillis.

D'autres caractéristiques et avantages de l'invention apparaîtront de la description détaillée qui va suivre d'un mode de réalisation préférentiel de l'invention, description faite en référence aux dessins annexés dans lesquels :
- la figure 1 représente sous forme de vue schématique les différentes étapes du procédé de l'invention ;
- la figure 2 est une vue en perspective de deux demi-boîtes constitutives d'une boîte d'incubation pour la mise en oeuvre du procédé ;
- la figure 3 est une vue en perspective d'une enceinte d'incubation ;
- la figure 4 est une vue simplifiée en coupe de cette même enceinte d'incubation ;
- la figure 5 est une vue en perspective d'une enceinte de nursery dépourvue de sa face avant et destinée à recevoir par glissement dans une rainure une demi-boîte constitutive d'une boîte d'incubation ;
- la figure 6 est une vue simplifiée en coupe de l'enceinte de nursery pourvue d'un fond formé par une demi-boîte.

Le procédé qui fait l'objet de l'invention comprend essentiellement quatre étapes qui consistent à séparer les oeufs du substrat dans lequel ils ont été pondus, à les placer dans des boîtes d'incubation dont au moins une grande face présente des ouvertures plus petites que le diamètre des oeufs, à placer ces boîtes à proximité immédiate d'une eau à niveau constant puis, lorsque les oeufs sont éclos, à ouvrir les boîtes et à les assembler avec une enceinte de nursery dépourvue de fond, de telle sorte que les grandes faces des boîtes forment après assemblage un fond pour l'enceinte de nursery.

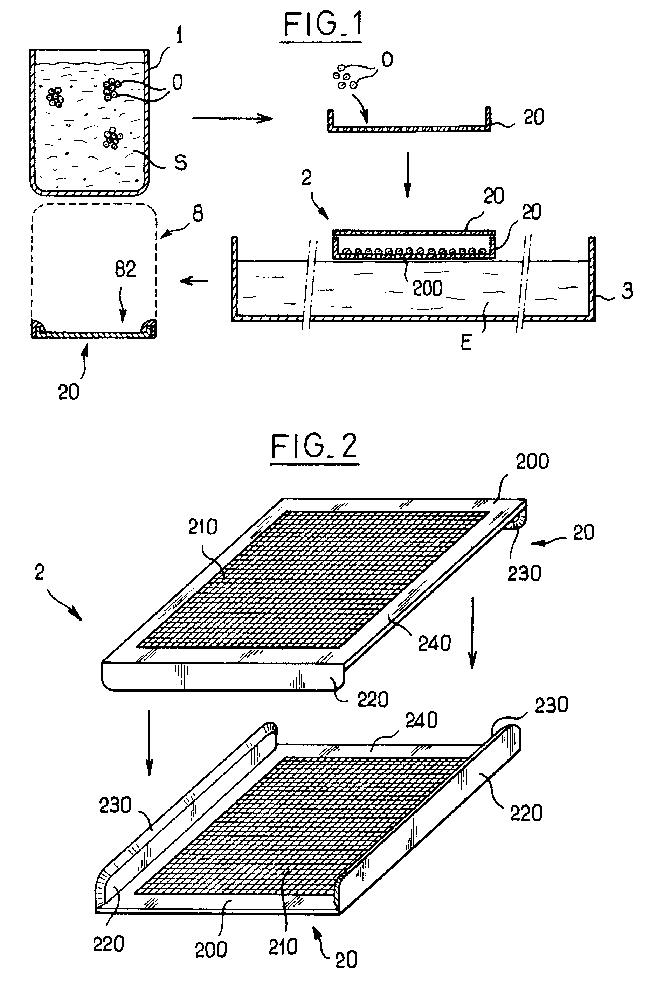

Avant d'expliquer en détail la mise en oeuvre de ce procédé, nous allons décrire les moyens préférés utilisés pour sa mise en oeuvre, plus spécifiquement en référence aux figures 2 à 6.

La boîte d'incubation 2 représentée à la figure 2 est formée de deux éléments identiques 20 formant demi-boîtes. Chaque élément 20 comporte une grande face 200 de forme carrée. Cette face est bordée sur deux de ses côtés opposés et perpendiculairement à celle-ci d'une paroi latérale 220 qui s'étend sur toute la longueur du côté et qui présente une hauteur faible. Cette paroi latérale est chanfreinée sur son côté tourné vers la grande face 200. Le chanfrein porte la référence 230.

Une grande partie de la grande face 200 est formée d'un treillis 210. Toutefois, ce treillis ne s'étend pas jusqu'au rebord de la face 200 mais est interrompu à une certaine distance de celui-ci pour former un pourtour plein 240.

De préférence, cette boîte d'incubation est obtenue par moulage d'une seule pièce d'une matière plastique.

A titre purement indicatif la dimension des côtés de la grande face 200 est d'environ 9 cm alors que les parois 220 présentent une hauteur de 8 mm.

Les ouvertures de mailles du treillis 210 sont d'environ 2 mm. Une telle dimension n'est en aucun cas limitative mais toutefois, il est particulièrement important que la dimension de ces ouvertures ne soient pas plus grande que le diamètre des oeufs d'escargots destinés à être mis en place sur le treillis.

L'obtention d'une boîte 2 à l'aide de deux demi-boîtes est obtenue simplement en superposant deux demi-boîtes tête-bêche, comme le montre la figure 2. L'emboîtement des deux éléments 20 se fait aisément, le chanfrein 230 permettant une mise en place et un assemblage aisés.

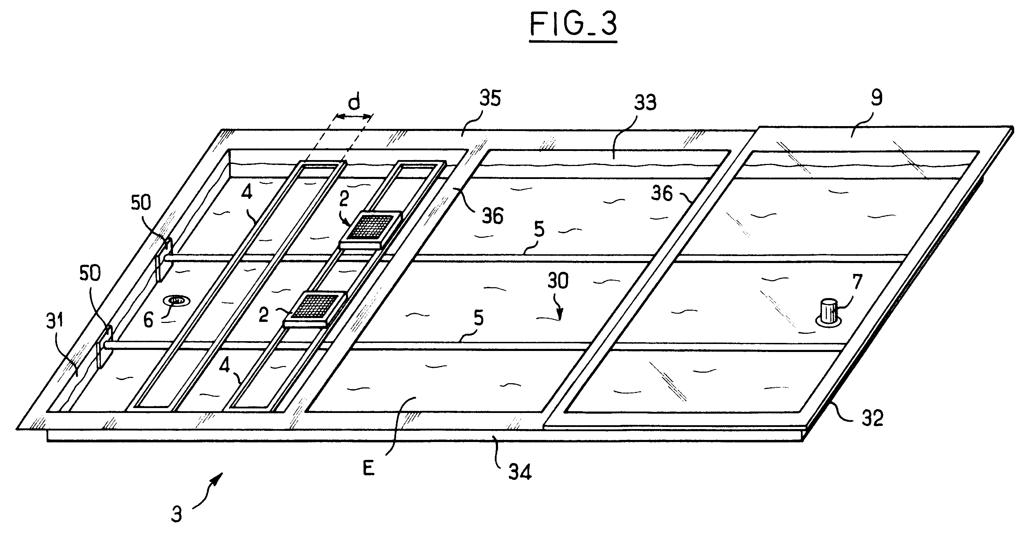

L'enceinte d'incubation, représentée en perspective à la figure 3, est formée d'un bac 3 de forme parallélépipèdique rectangle. Ce bac ouvert au niveau de sa face supérieure comporte un fond 30, deux petits côtés 31 et 32 ainsi que deux grands côtés 33 et 34. Ce bac est pourvu d'un rebord à angle droit 35 et de deux traverses 36 qui s'étendent parallèlement aux petits côtés du bac et qui délimitent, en quelque sorte, trois compartiments de surface égale.

Ce bac est réalisé par exemple en acier inoxydable ou en polyester.

Deux tiges supports 5, par exemple en acier inoxydable, s'étendent dans le sens longitudinal du bac, à une certaine distance du fond 30. Pour ce faire leurs extrémités sont solidaires de plaques de fixation 50 assujetties aux petits côtés 31 et 32 du bac.

A la figure 3 ont été représentés deux châssis 4 de forme générale rectangulaire, qui repose sur les tiges supports 5 et qui s'étendent donc dans le sens transversal du bac.

Ces châssis sont par exemple réalisés en matière plastique rigide.

L'écartement d entre les grands côtés de ces châssis est prévu pour que des boîtes d'incubation 2 puissent prendre appui sur ces châssis par leur grande face au niveau de leur pourtour dépourvu de treillis. De cette façon la totalité de la surface formée par le treillis se trouve en regard du fond du bac, comme le montre clairement la figure 3.

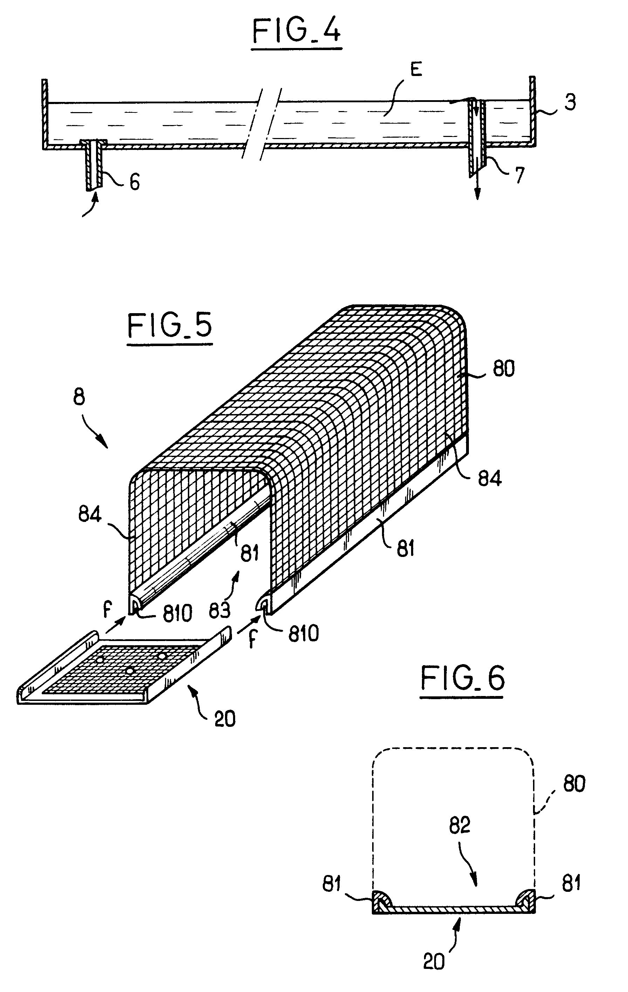

Ainsi que le montrent les figures 3 et 4, le bac 3 est équipé d'un conduit 6 qui traverse le fond du bac et débouche à l'intérieur de celui-ci. Ce conduit est destiné à l'alimentation en eau du bac. Sur ces figures, l'eau a été désignée par la référence E.

Par ailleurs, le bac est équipé d'un second conduit qui traverse également le fond 30 du bac qui débouche à une certaine distance de ce fond.

A la figure 4, on a figuré par des flèches l'écoulement de l'eau. Ainsi, lorsque l'arrivée d'eau par la conduite 6 est continue, le niveau d'eau E à l'intérieur du bac augmente jusqu'à ce qu'un trop plein s'écoule à travers le conduit 7 ; on est ainsi assuré d'avoir un niveau constant d'eau a l'intérieur de l'enceinte d'incubation.

De manière particulièrement importante, le positionnement des tiges supports 5 par rapport au fond 30 du bac et la hauteur d'eau E à l'intérieur de celui-ci sont choisis de telle manière que lorsque des boîtes d'incubation 2 sont en place sur les châssis 4, la surface de la grande face de la boîte tournée vers l'eau soit à une distance d'environ 1 mm de la surface de cette eau. On comprendra plus loin l'intérêt d'une telle caractéristique.

On a représenté à la figure 3 une vitre 9 en place sur une partie de l'enceinte d'incubation. Il peut être prévu, sur le rebord 35, un joint d'étanchéité par exemple en caoutchouc, ce qui permet, après mise en place de vitres 9, d'obtenir une fermeture parfaitement étanche de l'enceinte d'incubation. Sur cette figure, une seule vitre a été représentée, mais, bien entendu, la totalité de la surface du bac est destinée à en être équipée.

L'invention concerne également une enceinte de nursery pour la mise en oeuvre du procédé. Celle-ci est représentée en perspective à la figure 5.

Cette enceinte a une forme générale qui s'inscrit dans un parallélépipède rectangle. Il s'agit d'une boîte qui comporte un fond ouvert 83.

Il est particulièrement important de noter qu'à la figure 5, la petite face disposée vers l'avant de la figure et formant normalement la face frontale de l'enceinte, n'a pas été représentée pour des raisons de simplification et de compréhension.

Cette enceinte est formée d'un treillis 80 et de deux glissières 81 qui s'étendent le long des chants des grands côtés latéraux de la boîte.

On notera que cette boîte ne présente aucune partie anguleuse, que ce soit au niveau du treillis 80 que des glissières 81.

La face inférieure des deux glissières 81 est pourvue d'une rainure 810 de forme complémentaire de celle des parois latérales des demi-boîtes 20 qui forment les boîtes d'incubation 2 précédemment décrites.

L'écartement entre les deux glissières correspond sensiblement à la dimension des côtés des demi-boîtes 20. De même, la longueur des glissières 81 (c'est-à-dire de la boîte 8) correspond à un multiple de la longueur des côtés des demi-boîtes 20.

A la vue de la figure 5, on comprend qu'il est possible d'emmancher plusieurs demi-boîtes 20 dans les rainures 810 des glissières 81 afin de former un fond 82 (voir figure 6) pour cette enceinte de nursery.

Nous allons maintenant décrire la façon dont est mis en oeuvre le procédé de l'invention à l'aide du matériel décrit précédemment.

De manière générale, lorsque l'on procède à un élevage industriel d'escargots, la phase de ponte est mise en oeuvre alors que les escargots reproducteurs sont dans une enceinte fermée dans laquelle on a mis en place des pots de ponte référencés 1 à la figure 1. Il s'agit de pots d'une dizaine de centimètres de hauteur qui sont remplis d'un substrat S formé de terre, de sable ou d'un mélange de ceux-ci.

Les escargots viennent y pondre tour à tour. Les oeufs O se trouvant alors enfouis dans le substrat S sous forme de grappes.

Lorsque la phase de ponte est achevée, selon l'invention, on sépare les oeufs du substrat dans lequel ils ont été pondus. Pour ce faire on peut utiliser des moyens mécanisés. De préférence il est prévu un lavage des oeufs afin d'éliminer au maximum la présence de substrat S sur ces derniers. Bien entendu, cette phase de séparation est opérée avec minutie de manière à ne pas blesser les oeufs O qui sont relativement fragiles. De la même manière, on évite de toucher directement les oeufs afin de ne pas les contaminer.

Dans une deuxième étape, on place les oeufs nettoyés dans des boîtes d'incubation 2. Plus précisément, on place ces oeufs dans une demi-boîte 20, les oeufs O reposant alors sur le treillis 210. De manière générale, les oeufs d'escargots présentent un diamètre d'environ 3 mm de sorte que ceux-ci ne peuvent pas traverser les ouvertures du treillis. Lorsque les oeufs ont été placés sur la demi-boîte on forme alors une boîte d'incubation en superposant une seconde demi-boîte comme le montre la figure 2. Les oeufs se trouvent alors enfermés à l'intérieur de la boîte d'incubation.

Bien entendu, le nombre d'oeufs à mettre en place à l'intérieur de chaque boîte d'incubation est fonction des dimensions de celle-ci. Il s'agit d'une opération de mise en oeuvre à la portée de l'homme du métier.

Dans une troisième étape, on place les boîtes 2 contenant les oeufs sur les châssis 4 en place à l'intérieur de l'enceinte d'incubation 3. Au préalable, on a pris soin d'alimenter le bac en eau. De préférence, l'alimentation en eau est continue pendant toute la période d'incubation des oeufs de manière à ce que s'opère un écoulement continu du trop plein d'eau par le conduit 7. Il peut être prévu sur les châssis 4 des ergots (non représentés) facilitant le positionnement des boîtes.

Il est bien connu que l'éclosion des oeufs d'escargots est favorisée par une température de l'ordre de 25 à 26° et une hygrométrie du milieu ambiant environnant les oeufs de 90 %.

Dans le but d'atteindre ces paramètres, on utilise une eau dont la température est de cet ordre. Il peut s'agir d'une eau préalablement chauffée ; il peut éventuellement être prévu, au niveau du fond du bac, des moyens de chauffage de l'eau.

Afin de maintenir une hygrométrie convenable à l'intérieur de l'enceinte d'incubation, on place sur le bac plusieurs plaques 9 de maniere à rendre celui-ci étanche. Ainsi, à l'intérieur de l'enceinte on peut maintenir une température convenable et éviter l'évaporation de l'eau.

Du fait que la distance entre la surface de l'eau et les boîtes d'incubation est très petite (de l'ordre de 1 mm comme mentionné ci-dessus), les oeufs se trouvent dans un milieu propice à leur éclosion qui intervient généralement au bout de quinze jours à trois semaines.

Toutefois, il est important que l'eau ne touche pas à la surface des boîtes. En effet, si tel était le cas, l'eau remontrait par capillarité à l'intérieur des boîtes en provoquerait alors la mort des oeufs par noyade.

Le matériau dans lequel est réalisé le bac 3 (acier inoxydable ou polyester) est relativement important dans la mesure ou il ne doit se corroder et ne pas favoriser le développement de bactéries.

L'écoulement continu de l'eau par les conduites 6 et 7 provoque un renouvellement constant de celle-ci et permet ainsi l'élimination des germes et bactéries qui pourraient se trouver dans l'eau.

Lorsque les oeufs sont éclos, on ouvre alors les boîtes d'incubation 2. Les jeunes escargots, qui ont alors une dimension de quelques millimètres se déplacent sur les parois des demi-boîtes 20.

On transfère alors les escargots dans des enceintes de nursery telles que celle représentée à la figure 5.

Comme on l'a précisé plus haut, les jeunes escargots sont très sensibles au stress et aux contaminations, il est donc important de pouvoir opérer un transfert de ces derniers dans les enceintes de nursery sans avoir à les manipuler manuellement.

Une telle opération est rendue possible grâce au fait qu'il suffit de saisir les demi-boîtes 20 sur lequel sont présents les escargots par les parois latérales et à faire glisser ces demi-boîtes le long des nervures 810 des glissières 80 de l'enceinte. Lorsque le nombre adéquat de demi-boîtes a été mis en place on a alors une enceinte de nursery 8 complètement fermée, a l'intérieur de laquelle se trouve les escargots.

Le stress des animaux a été réduit au minimum du fait qu'aucune intervention manuelle n'a été faite sur eux. De la même manière le risque de contamination de ceux-ci est réduit. Le taux de mortalité se trouve donc réduit considérablement.

Des enceintes de nursery du type de celle représentée à la figure 5 peuvent être alimentées en farine d'engraissement et en eau (pour l'alimentation et le nettoyage des enceintes) par des moyens automatiques. Pour ce faire, les enceintes de nursery peuvent être disposées sur des supports grillagés équipés de ces moyens automatiques de fourniture d'eau et d'aliments.

Selon une variante de réalisation, les conduits 6 et 7 d'amenée d'évacuation de l'eau dans l'enceinte d'incubation ont une forme en col de cygne.

A titre indicatif, l'enceinte d'incubation présente les dimensions suivantes : longueur 1 m 45, profondeur : 4 cm, largeur : 60 cm.

Egalement à titre purement indicatif, une enceinte de nursery conforme à l'invention présente une hauteur d'environ 10 cm. Sa largeur, au niveau des rainures 810 est de l'ordre de celle des demi-boîtes d'incubation, (pour la demi-boîte représentée à la figure 2, cette dimension est de 9 cm). La longueur de cette enceinte est par exemple de 36 cm, c'est-à-dire qu'elle peut recevoir quatre demi-boîtes côte à côte pour en former le fond.

## Revendications

1. Procédé d'incubation d'oeufs d'escargots (O), qui consiste à séparer les oeufs (O) du substrat (5) dans lequel ils ont été pondus, à les placer dans des boîtes d'incubation (2) dont au moins une grande face (200) présente des ouvertures de dimensions plus petites que le diamètre des oeufs, à placer lesdites boîtes à proximité immédiate d'une eau (E) à niveau constant, la face (200) présentant des ouvertures étant tournée vers ladite eau, puis, lorsque les oeufs sont éclos, à ouvrir lesdites boîtes (2) et à les assembler avec une enceinte de nursery (8) dépourvue de fond, de telle sorte que les grandes faces (200) des boîtes forment, après assemblage, un fond (82) pour l'enceinte de nursery (8).

2. Procédé selon la revendication 1, caractérisé en ce que, lorsqu'on place les boîtes à proximité de ladite eau, la distance qui sépare la face (200) de la boîte (2) tournée vers l'eau (E) de cette dernière, est d'environ 1 mm.

3. Boîte d'incubation pour la mise en oeuvre du procédé selon les revendications 1 et 2, caractérisée en ce qu'elle consiste en deux éléments identiques (20) formant demi-boîtes, lesquels comportent une grande face (200) de forme carrée pourvue, sur deux de ses côtés opposés et perpendiculairement à celle-ci, d'une paroi latérale (220).

4. Boîte selon la revendication 3, caractérisée en ce que ladite paroi latérale (200) est chanfreinée sur son côté tourné vers la grande face (200).

5. Boîte selon l'une des revendications 3 et 4, caractérisée en ce qu'au moins une partie de la surface de ladite grande face (200) est formée d'un treillis (210).

6. Enceinte d'incubation pour la mise en oeuvre du procédé selon l'une des revendications 1 et 2, caractérisée en ce qu'elle a la forme d'un bac (3) qui est équipé de moyens de circulation d'eau et de maintien à niveau de celle-ci (6, 7) et en ce qu'elle est pourvue de moyens supports (4) pour les boîtes d'incubation.

7. Enceinte selon la revendication 6, caractérisée en ce que les moyens de maintien à niveau de l'eau consistent en un conduit (7) sensiblement vertical dont l'orifice supérieur débouche à une certaine distance du fond (30) du bac (3) et qui assure l'évacuation du trop plein d'eau.

8. Enceinte d'incubation selon l'une des revendications 6 et 7, caractérisée en ce que lesdits moyens supports consistent en des châssis (4) de forme rectangulaire sur lesquels lesdites boîtes (2) sont destinees à reposer par deux de leurs côtés opposés, et qui s'étendent parallèlement à l'un des côtés (31) de l'enceinte, ces cadres étant disposés de telle façon que, lorsque les boîtes sont en place, celles-ci se trouvent à proximité immédiate de l'eau.

9. Enceinte selon la revendication 8, caractérisée en ce que lesdits cadres reposent sur des tiges supports (5).

10. Enceinte selon l'une des revendications 6 à 9, caractérisée en ce qu'elle est pourvue de moyens de fermeture hermétiques (9).

11. Enceinte de nursery pour la mise en oeuvre du procédé selon l'une des revendications 1 et 2, caractérisée en ce qu'elle est formée d'une boîte (8) qui comporte un fond ouvert (83) de forme rectangulaire et en ce que les chants (81) des deux grandes faces opposées (84) de ladite boîte adjacents au fond ouvert (83) présentent une rainure (810) de forme complémentaire de celle des parois latérales (220) de la boîte d'incubation (2) conforme à l'une des revendications 3 à 5, les dimensions du fond ouvert étant telles que l'on peut mettre en place, côte-à-côte et par glissement dans ladite rainure (810) plusieurs demi-boîtes (20) constituant les boîtes d'incubation (2), de façon à former un fond (82) pour l'enceinte de nursery.

12. Enceinte de nursery selon la revendication 11, caractérisée en ce que ses parois ne comportent aucun angle vif.

13. Enceinte de nursery selon l'une des revendications 11 et 12, caractérisée en ce qu'elle est formée, au moins en partie, d'un treillis (80).
